# EUROPEAN PATENT SPECIFICATION

(11) **EP 2 611 403 B1**
(45) Date of publication and mention of the grant of the patent: **14.12.2016**
(21) Application number: 11821907.0
(22) Date of filing: 25.08.2011
(51) Int. Cl.: A61F 13/00, A61F 13/15, A61F 13/53, A61F 13/56, A61F 13/532

(54) **INCONTINENCE LINER**
INKONTINENZAUSKLEIDUNG
COUCHE-CULOTTE D'INCONTINENCE

(30) Priority: 31.08.2010 JP 2010194203
(43) Date of publication of application: 10.07.2013
(73) Proprietor: Unicharm Corporation, Ehime 799-0111 (JP)
(72) Inventor: KAWAKAMI, Yusuke, Kanonji-shi Kagawa 769-1602 (JP)
(74) Representative: Dolleymores
(86) International application number: PCT/JP2011/069861
(87) International publication number: WO 2012/029892

(56) References cited:
- EP-A1- 2 281 536
- JP-A- 2001 190 581
- JP-A- 2006 230 714
- JP-A- 2008 178 667
- JP-A- 2009 056 269
- JP-A- 2009 100 846
- US-A1- 2006 058 762

## Description

### Technical Field

The present invention disclosure relates to an incontinence liner.

### Background Art

Known in the art is a sanitary napkin provided with a liquid permeable skin contact side sheet, a liquid impermeable and moisture permeable non-skin contact side sheet, and an absorptive body which is arranged between these skin contact side sheet and non-skin contact side sheet (see PLT 1). The sanitary napkin is provided with a pair of wings for fastening the napkin to clothing, the wings being provided adjoining an intermediate region of the absorptive body and being bent and superposed over the intermediate region when worn. In this napkin, the napkin is reliably fastened to the clothing by the wings. Further, by using the moisture-permeable non-skin contact side sheet, dampness is suppressed. EP 2281536 (PLT 2) discloses an absorbent article which comprises an absorber and heat absorbing material provided inside this absorber. As PLT 2 was published after the priority date of the present application, it only forms prior art under Article 54(3) EPC.

### Citation List

### Patent Literature

PLT 1: Japanese Patent Publication (A) No. 2003-088550
PLT 2: European Patent Application EP 2281536 A1

### Summary of Invention

### Technical Problem

As explained above, in relation to the sanitary napkin described in PLT 1, the wings are superposed over the intermediate region of the absorptive body when the sanitary napkin is worn. As a result, at the intermediate region, at the outer side of the absorptive body, the non-skin contact side sheet, clothing, and wings are successively superposed. Therefore, the air permeability at the intermediate region might fall which, in turn, might cause unpreferable dampness to occur. If large dampness occurs, a rash might occur on the skin of the wearer. In general, the frequency of changing the incontinence liner is lower than that of a sanitary napkin, and therefore the wearing time of the liner is longer. Therefore, in the case of an incontinence liner, a rash is more likely to occur than in the case of a sanitary napkin.

### Solution to Problem

The present invention provides the incontinence liner of independent Claim 1. The dependent claims specify preferred but optional features.

According to the present invention, there is provided an incontinence liner provided with a liquid permeable skin contact side sheet, a liquid impermeable and moisture permeable non-skin contact side sheet, and an absorptive body arranged between these skin contact side sheet and non-skin contact side sheet, in which liner, the absorptive body includes a front region, a rear region, and an intermediate region between these front region and rear region, the liner is provided with a pair of wings for fastening the liner to clothing, which wings being provided adjoining the intermediate region and being bent and superposed over the intermediate region when worn, the absorptive body includes a hygroscopic material, and a basis weight of the hygroscopic material in the intermediate region is larger than a basis weight of the hygroscopic material at the front region and a basis weight of the hygroscopic material at the rear region, wherein said liner is provided with adhesives applied to an outer surface of said non-skin contact side sheet and extending separated from each other in a width direction, and extending in parallel in a longitudinal direction and compression grooves extending from said intermediate region up to said front region and rear region, said adhesives being positioned at the two sides around the front edges of said compression grooves in the width direction and extending beyond the front edges toward the front in the longitudinal direction and being positioned at the two sides around the rear edges of said compression grooves in the width direction and extending over the rear edges to the rear in the longitudinal direction.

### Advantageous Effects of Invention

It is possible to reliably suppress dampness in the incontinence liner.

### Brief Description of the Drawings

FIG. 1 is a plan view of an incontinence liner.
FIG. 2 is a longitudinal cross-sectional view of the liner taken along line M-M of FIG. 1.
FIG. 3 is the back view of an incontinence liner.
FIG. 4 is a plan view of an absorptive body of the incontinence liner.
FIG. 5 is a schematic longitudinal cross-sectional view of the liner when worn.
FIG. 6 is a partial schematic longitudinal cross-sectional view of parts of the absorptive body.
FIG. 7 is a partial schematic longitudinal cross-sectional view of a compression groove in the absorptive body.
FIG. 8 is a schematic side cross-sectional view of a package containing the liner.

### Description of Embodiments

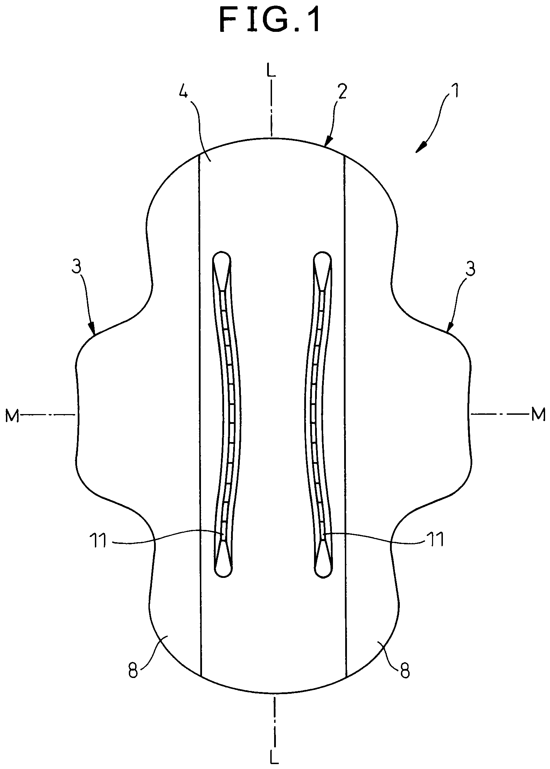

Referring to FIG. 1, the incontinence liner 1 is provided with an oval-shaped main body 2 and a pair of wings 3 which project outwardly from the side parts of the main body 2 in the width direction. The present invention is not to be limited to this particular shape of main body.

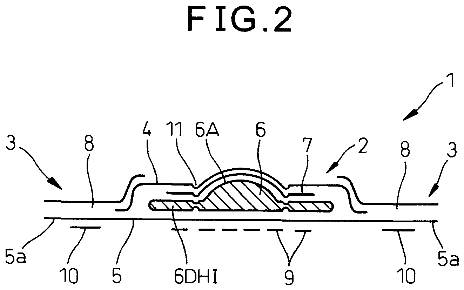

In particular, as shown in FIG. 2, the main body 2 is comprised of a skin contact side sheet (skin contacting sheet) 4 and a non-skin contact side sheet (non-skin contacting sheet) 5 superposed on each other, an absorptive body 6 arranged between these skin contact side sheet 4 and non-skin contact side sheet 5, and a cushion sheet 7 arranged between the skin contact side sheet 4 and the absorptive body 6. On the other hand, the wings 3 are comprised of parts 5a of the non-skin contact side sheet 5 extending outwardly beyond the side edges of the skin contact side sheet 4 in the width direction and side sheets 8 superposed on the parts 5a. Here, the skin contact side sheet 4 and non-skin contact side sheet 5 and the non-skin contact side sheet parts 5a and side sheets 8 are respectively joined by for example a hot melt adhesive, heat sealing, etc. Note that, in an embodiment of the present invention, the side sheets 8 are also superposed and joined with the skin contact side sheet 4, and therefore, the main body 2 also includes the side sheets 8. In further embodiments, the non-skin contact side sheet 5 may also not extend up to the wings 3. In this case, the wings 3 may be comprised of only the side sheets 8, or the side sheets 8 and other sheets superposed with the same.

In an embodiment according to the present invention, the absorptive body 6 is joined with the cushion sheet 7 and non-skin contact side sheet 5 by a hot melt adhesive. In this case, the hot melt adhesive is applied in a mesh shape. As a result, the hot melt adhesive is kept from causing the air permeability to fall.

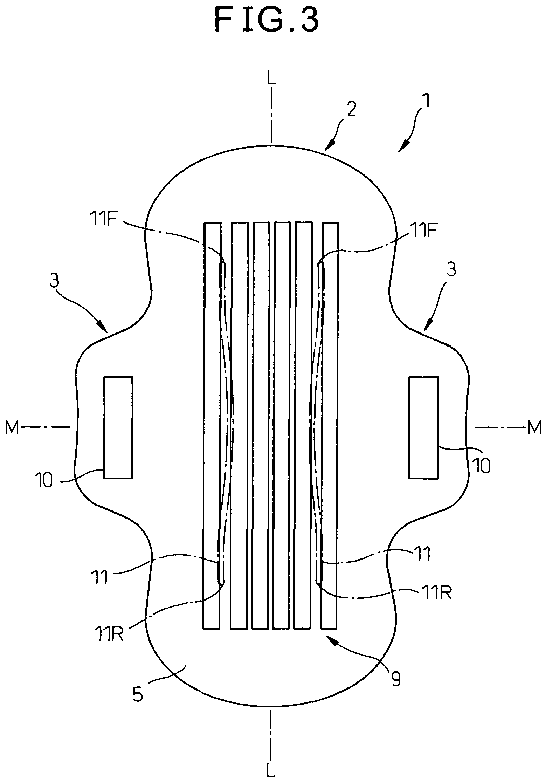

Further, as shown in FIG. 2 and FIG. 3, at the outer surface of the non-skin contact side sheet 5 corresponding to the back surface of the main body 2, adhesives 9, e.g., adhesive stripes, separated from each other in the width direction and extending in parallel in the longitudinal direction are applied, while at the outer surface of the non-skin contact side sheet 5 corresponding to the back surfaces of the wings 3, adhesives 10 are applied.

Further, the liner 1 is formed with a pair of compression grooves 11 extending from the skin contact side sheet 4 to the absorptive body 6. In an embodiment according to the present invention, each of the compression grooves 11 is comprised of deep groove sections and shallow groove section, which alternately repeat in the length (longitudinal) direction. In some embodiments, the compression grooves 11 may also be comprised of deep grooves which extend continuously in the length direction.

Note that, in an embodiment according to the present invention, the liner 1 is formed symmetrically about a longitudinal direction center line L-L and a width direction center line M-M. In some embodiments, the liner 1 is formed symmetrically only about the longitudinal direction center line L-L.

The skin contact side sheet 4 has liquid permeability and is, for example, comprised of a woven fabric or nonwoven fabric made of polyethylene (PE), polypropylene (PP), polyethylene terephthalate, or other polyolefin-based thermoplastic hydrophobic fiber treated to make it hydrophilic or is comprised of pulp, cotton, or other natural fiber or rayon or another cellulose fiber. In an embodiment according to the present invention, as the skin contact side sheet 4, a 20 to 40 g/m² air-through nonwoven fabric comprised of PE/PP fiber is used.

The non-skin contact side sheet 5 has liquid impermeability and moisture permeability and is, for example, comprised of a hydrophobic nonwoven fabric, a water impermeable plastic film, a laminate sheet of a nonwoven fabric and water impermeable plastic film, a high waterproof melt blown nonwoven fabric, or an SMS (spun bonded/melt blown/spun bonded) nonwoven fabric having a high strength spun bonded nonwoven fabrics sandwiching therebetween a melt blown nonwoven fabric. In an embodiment according to the present invention, as the non-skin contact side sheet 5, a 20 to 35 g/m² or so moisture permeable PE film is used.

The absorptive body 6 has liquid retention ability and is comprised of an absorbent core. The absorbent core includes an absorptive material and a hygroscopic material. Note that in an embodiment according to the present invention, the absorptive body 6 is wrapped by thin paper or a liquid permeable spun bonded nonwoven fabric, SMS nonwoven fabric, etc.

The absorptive material, for example, is comprised of a fluff-like pulp or air laid nonwoven fabric. Here, the fluff-like pulp is, for example, comprised of chemical pulp, cellulose fiber, rayon, acetate, or other artificial cellulose fiber, while the air laid nonwoven fabric is, for example, comprised of pulp and synthetic fiber melt bonded or fixed by a binder to form a nonwoven fabric.

On the other hand, the hygroscopic material preferably does not contain any ingredients harmful or irritating to the human body. In particular, when the hygroscopic material includes ingredients which can dissolve in water, the ingredients are likely to dissolve and contact the skin when the ingredients contact urine etc., so the hygroscopic material is preferably insoluble in water in practice. When the hygroscopic material is water soluble, it is preferably treated to make it insoluble.

Specifically, the hygroscopic material is, for example, comprised of a granular high absorption polymer (or super absorption polymer, SAP). The SAP is, for example, comprised of a sodium polyacrylate cross-linked product, a starch-polyacrylate graft polymer, hydrophilic polyurethane, a copolymer of a monomer having a carboxylate, sulfonate, or sulfate group and a monomer having a phosphate or a phosphonate group, or other high absorption polymer. The hygroscopic material may also be comprised of inorganic/organic porous substances which physiochemically adsorb moisture on their surfaces such as activated carbon, natural or synthetic silica, and synthetic zeolite. Alternatively, it is possible to comprise the hygroscopic material from potassium chloride or other substances which take in moisture as the water of crystallization.

Here, if the mass ratio of the mass of the SAP to the mass of the absorbent core, that is, to the total mass of the pulp and the SAP, is called the "SAP mass ratio", in an embodiment according to the present invention, the SAP mass ratio is set to 30 to 70 mass percent and, preferably, 35 to 55 mass percent. If the SAP mass ratio is smaller than 30 mass percent, it becomes difficult to secure an absorptive material density sufficient for imparting liquid diffusibility. If the SAP mass ratio is larger than 70 mass percent, the pulp or another fiber ingredient becomes relatively insufficient, a gel blocking phenomenon of the high absorption polymer easily occurs, and therefore the liquid diffusibility is likely to fall or, when worn, the absorptive body is likely to lose its shape due to the external force acting on the absorptive body. Note that the SAP mass ratio of a sanitary napkin is generally 0 to 10 mass percent, at most 20 mass percent, so the incontinence liner according to the present invention differs in constitution from a sanitary napkin in this respect.

The cushion sheet 7 is, for example, comprised of a nonwoven fabric made of a polyolefin-based thermoplastic hydrophobic fiber treated to be made hydrophilic. As the nonwoven fabric, an air-through nonwoven fabric, spun bonded nonwoven fabric, SMS nonwoven fabric, etc. may be used. In an embodiment according to the present invention, as the cushion sheet 7, a 20 to 40 g/m² air-through nonwoven fabric comprised of PE/PP fiber is used.

The side sheets 8 are, for example, comprised of a nonwoven fabric of a polyolefin-based thermoplastic hydrophobic fiber. As the nonwoven fabric, an air-through nonwoven fabric, spun bonded nonwoven fabric, SMS nonwoven fabric, etc. may be used. In an embodiment according to the present invention, as the side sheets 8, a 15 to 35 g/m² air-through nonwoven fabric comprised of PE/PP fibers is used.

The adhesives 9 and 10 are, for example, comprised of a styrene-isoprene-styrene block copolymer (SIS), styrene-butadiene-styrene block copolymer (SBS), styrene-ethylene-butylene-ethylene copolymer (SEBS), or another hot melt adhesive.

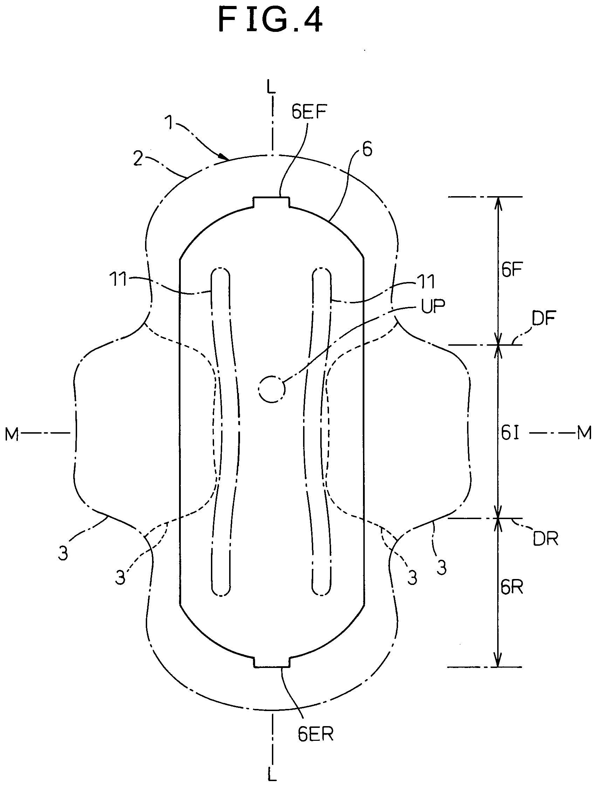

The absorptive body 6, as shown in FIG. 4, includes regions defined with respect to each other by defining (dividing) lines DF and DR extending in the width direction, that is, a front region 6F, a rear region 6R, and an intermediate region 6I between the front region 6F and the rear region 6R. In this case, the front region 6F is defined between a front edge 6EF of the absorptive body 6 and front defining line DF, the intermediate region 6I is defined between the front defining line DF and a rear defining line DR, and the rear region 6R is defined between the rear defining line DR and a rear edge 6ER of the absorptive body 6. Note that, in an embodiment according to the present invention, the absorptive body 6 is formed symmetrically about the longitudinal direction center line L-L and the width direction center line M-M. In some embodiments, the absorptive body 6 is formed symmetrically only about the longitudinal direction center line L-L.

In an embodiment according to the present invention, the absorptive body 6 is formed so that the basis weight of the hygroscopic material at the intermediate region 6I is larger than the basis weight of the hygroscopic material at the front region 6F and the basis weight of the hygroscopic material at the rear region 6R.

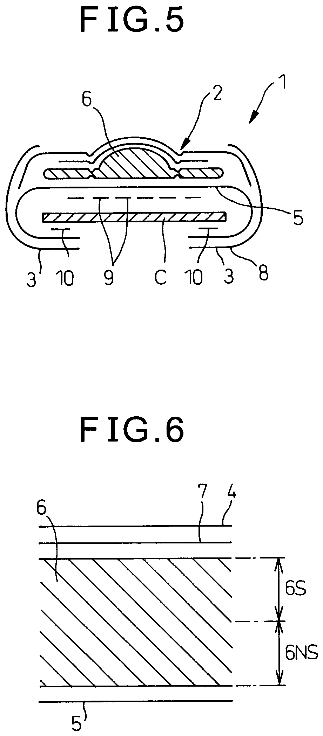

When worn, as shown in FIG. 5, the main body 2 is fixed by the adhesives 9 to the inner surface of clothing C such as underwear of the wearer, while the wings 3 are fixed to the outer surface of the clothing C by the adhesives 10. That is, the main body 2 and wings 3 sandwich the clothing between them whereby the liner 1 is fastened to the inner side of the clothing C. As a result, wrinkles or twisting can be kept from occurring at the liner 1 when worn, therefore the liner 1 can continue to be reliably fastened to the clothing C over a long period of time. Note that, the clothing to which the liner 1 is fastened generally is panty-shaped with an elastic torso part and elastic leg parts. By fitting over the torso and legs of the wearer, the clothing as a whole fits against the skin of the wearer. As a result, the liner 1 fastened to the inner side of the clothing also is worn in a state fit along the skin of the wearer extending in the front-back direction centered at the crotch part.

In this case, the liner 1 is fastened at the crotch part of the clothing C at the substantial center in the front-back direction. As a result, the urinating position UP of the wearer (FIG. 4) becomes positioned at the intermediate region 6I of the absorptive body 6, in particular, at a position in the intermediate region 6I near the front region 6F. Therefore, the liquid to be absorbed mainly comprised of urine is mainly absorbed at the intermediate region 6I. Next, the liquid to be absorbed diffuses to the front region 6F and the rear region 6R.

Referring again to FIG. 4, the above-mentioned wings 3 are positioned adjoining the intermediate region 6I of the absorptive body 6. For this reason, when worn, the bent wings 3 are superposed over the intermediate region 6I of the absorptive body 6 as shown by the broken line in FIG. 4. Note that it is also possible to see this as the intermediate region 6I being partitioned so as to include parts of the absorptive body 6 where the bent wings 3 are superposed.

As a result, at the intermediate region 6I, as will be understood from FIG. 5, at the outer side of the absorptive body 6, the non-skin contact side sheet 5, the clothing C, and the wings 3 are successively superposed. Therefore, the air permeability at the intermediate region 6I might fall and unpreferable dampness might occur.

However, in an embodiment according to the present invention, the basis weight of the hygroscopic material at the intermediate region 6I is made larger than the basis weight of the hygroscopic material at the front region 6F and the basis weight of the hygroscopic material at the rear region 6R. As a result, it is possible to suppress dampness at the intermediate region 6I. This means that it is unlikely that the wearer will have an unpleasant feeling to the wearer even though the liner 1 is worn for a long time period.

When comprising the hygroscopic material from SAP granules, the SAP granules are, for example, preferably of a size passable through a 300 µm mesh sieve. This is because small granules, as compared with large granules, have a larger specific surface area (total surface areas of granules per unit mass), so as to be superior in hygroscopicity. Further, if using small size SAP granules in this way, the touch to the skin of the wearer is improved. On the other hand, the SAP granules are preferably, for example, of a size not passable through a 150 µm mesh sieve. This is because if the SAP granules become smaller in size, not only does the hygroscopic performance fall, but also the blocking phenomenon is likely to occur.

Table 1 shows test results showing differences in hygroscopic rates due to the sizes of the SAP granules. In this experiment, 1 g amounts of SAP of predetermined sizes were placed in 10 mm SUS Petri dishes and were allowed to stand under conditions of 35°C and 75% RH (relative humidity) for predetermined times. After that, the masses of the SAP were measured and the hygroscopic rates were calculated (hygroscopic rate=(mass of SAP after standing - mass of SAP before standing)/(mass of SAP before standing)). Table 1 shows that in the case of a standing time of 4 hours, if the SAP granules were of a size passable through a 300 µm mesh sieve, there would be a hygroscopic rate (degree) higher than 50 percent.

**[Table 1]**

| Grain (granule) size | Standing time (hours) | | | | | |
|---|---|---|---|---|---|---|
| | 0 | 1 | 1.5 | 2 | 3 | 4 |
| Passable through 150 µm mesh sieve | 0 | 14 | 32 | 43 | 48 | 50 |
| Not passable through 150 µm mesh sieve, but passable through 250 µm mesh sieve | 0 | 12 | 30 | 42 | 46 | 51 |
| Not passable through 250 µm mesh sieve, but passable through 300 µm mesh sieve | 0 | 12 | 29 | 43 | 48 | 52 |
| Not passable through 300 µm mesh sieve, but passable through 500 µm mesh sieve | 0 | 8 | 26 | 39 | 43 | 47 |
| Not passable through 500 µm mesh sieve, but passable through 710 µm mesh sieve | 0 | 9 | 25 | 39 | 42 | 45 |
| Not passable through 710 µm mesh sieve | 0 | 11 | 27 | 39 | 43 | 45 |

| | | | | | | |
|---|---|---|---|---|---|---|
| (unit: %) | | | | | | |

In an embodiment according to the present invention, the absorptive body 6 is formed so that the mass ratio of the part of the SAP granules passable through a mesh 300 µm sieve in the absorptive body 6 (=(the mass of the part of the SAP granules passable through a mesh 300 µm sieve in the absorptive body 6)/(the total mass of the SAP granules included in the absorptive body 6)) is at least 50 percent and, preferably, 50 to 75 percent. With this preferred embodiment, it is possible to suppress the blocking phenomenon while securing an excellent hygroscopic action.

Further, in an embodiment according to the present invention, the absorptive body 6 is formed so that the mass ratio of the part of the SAP granules passable through a mesh 300 µm sieve in the intermediate region 6I (=(the mass of the part of the SAP granules passable through a mesh 300 µm sieve in the intermediate region 6I)/(the total mass of the SAP granules included in the intermediate region 6I)) is higher than the mass ratio of the part of the SAP granules passable through a mesh 300 µm sieve in the front region 6F (=(the mass of the part of the SAP granules passable through a mesh 300 µm sieve in the front region 6F)/(the total mass of the SAP granules included in the front region 6F)) and the mass ratio of the part of the SAP granules passable through a mesh 300 µm sieve in the rear region 6R (=(the mass of the part of the SAP granules passable through a mesh 300 µm sieve in the rear region 6R)/(the total mass of the SAP granules included in the rear region 6R)). With this preferred embodiment, it is possible in particular to suppress dampness in the intermediate region where the wings 3 are superposed.

Furthermore, if considering the fact that the absorptive body 6, as shown in FIG. 6, includes the skin contact side region 6S and non-skin contact side region 6NS, the absorptive body 6 is formed so that the mass ratio of the part of the SAP granules passable through a mesh 300 µm sieve in the skin contact side region 6S (=(the mass of the part of the SAP granules passable through a mesh 300 µm sieve in the skin contact side region 6S)/(the total mass of the SAP granules included in the skin contact side region 6S)) is higher than the mass ratio of the part of the SAP granules passable through a mesh 300 µm sieve in the non-skin contact side region 6NS (=(the mass of the part of the SAP granules passable through a mesh 300 µm sieve in the non-skin contact side region 6NS)/(the total mass of the SAP granules included in the non-skin contact side region 6NS)). With this preferred embodiment, it is possible to arrange small size SAP granules nearer to the wearer, possible to reliably suppress dampness, and simultaneously possible to suppress an odd feeling due to the presence of the SAP granules.

The SAP granules used as the hygroscopic material are produced using, for example, the inverse phase suspension polymerization method or the aqueous solution polymerization method. In this case, if using the inverse phase suspension polymerization method, the primary particle size of the SAP granules becomes smaller, so the inverse phase suspension polymerization method is preferably used. This is because if the primary particle size of the SAP granules becomes smaller, the specific surface area becomes larger and the hygroscopicity is improved.

Here, the method of measurement of the particle size distribution of the SAP granules included in the different parts 6F, 6I, and 6R of the absorptive body 6 in an embodiment according to the present invention will be briefly explained. First, sieves of meshes of 710, 500, 300, 250, and 150 µm were prepared and the masses of the sieves are measured. These sieves were stacked in order of mesh size with the smallest meshes first, and set in a shaker machine. On the other hand, wrapping thin paper etc. was removed from the parts 6F, 6I, and 6R being measured and the absorbent cores were taken out. The pulp was removed from the absorbent cores and the SAP is taken out. The taken out SAP was measured for mass and placed in the topmost (largest mesh size) sieve. Next, the shaker machine was operated to shake the assembly for 30 minutes. Next, the masses of the sieves were measured and the masses of just the sieves measured in advance were subtracted to obtain the masses of the SAP granules remaining at the different sieves. Next, the ratios of the masses of the SAP granules remaining at the different sieves to the mass of the SAP granules as a whole taken out from the absorbent core were calculated. These were used to find the particle size distribution of the SAP granules.

Referring again to FIG. 4, the above-mentioned compression grooves 11 extend from the intermediate region 6I up to the front region 6F and the rear region 6R. As a result, the absorptive body 6 can be bent along the compression grooves 11, and the liner 1 can deform to track the body of the wearer. Further, the compression grooves 11 are relatively high in absorptive material density, so the liquid to be absorbed diffuses inside of the compression grooves 11 as well to reach the front region 6F and the rear region 6R where it is further diffused.

Further, the compression grooves 11 can also serve as air passages. That is, moisture in the intermediate region 6I can pass through the compression grooves 11 to the front region 6F and the rear region 6R and reach the outside of the liner 1. Therefore, it is possible to further suppress dampness.

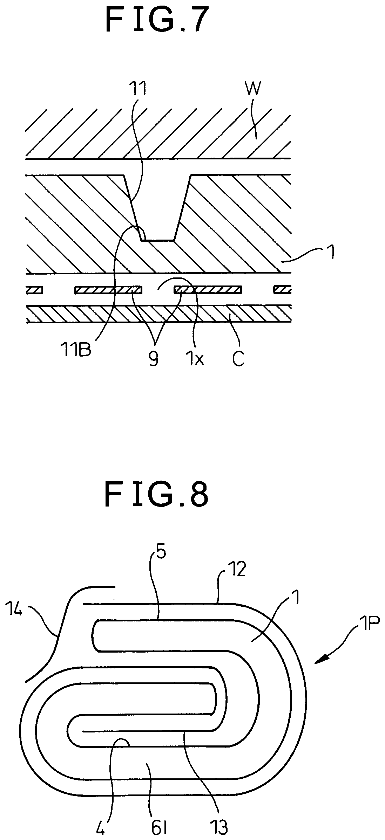

Furthermore, if referring to FIG. 7 and FIG. 3, the adhesives 9 are positioned at the two sides of the areas around the front edges 11F of the compression grooves 11 in the width direction and extend beyond the front edges 11F toward the front in the longitudinal direction. Similarly, the adhesives 9 are positioned at the two sides of the areas around the rear edges 11R of the compression grooves 11 in the width direction and extend beyond the rear edges 11R to the rear in the longitudinal direction.

By this embodiment, the areas around the front edges 11F and the areas around the rear edges 11R of the compression grooves 11 are surrounded by the two adhesives 9. As a result, even if the liner 1 is bent along the compression grooves 11, the shapes of the compression grooves 11 are maintained. That is, even if the liner 1 is deformed, the air passage action of the compression grooves 11 is maintained. Therefore, it is possible to reliably suppress dampness over a long period of time.

More particularly, at the intermediate region 6I positioned around the crotch surrounded by the two legs of the wearer, sometimes the movement of the legs of the wearer causes a force to act in a direction compressing the liner in the width direction and resulting in the liner 1 deforming. On the other hand, the areas around the front edges 11F and around the rear edges 11R of the compression grooves 11 are positioned away from the crotch of the wearer in the longitudinal direction or front-back direction. In this respect, if the above-mentioned force in the width direction is conveyed to the areas around the front edges 11F and around the rear edges 11R of the compression grooves 11, the compression grooves 11 are likely to deform and shut. However, in an embodiment according to the present invention, as described above, the adhesives 9 are provided, so deformation of the areas around the front edges 11F and around the rear edges 11R of the compression grooves 11 is suppressed. Therefore, it is possible to reliably allow moisture to escape from around the intermediate region 6I where the humidity will become the highest, through the compression grooves 11 to the front region 6F and the rear region 6R.

On the other hand, the adhesives 9 do not overlap the areas around the front edges 11F and rear edges 11R of the compression grooves 11, in particular the bottoms 11B. That is, the outer surfaces of the liner 1 or non-skin contact side sheet 5 facing the compression grooves 11, in particular their bottoms 11B, are provided with adhesive-free regions 1x. As a result, a deformation of the liner 1 along with compression grooves 11 is not inhibited by the adhesives 9. Therefore, when the liner 1 deforms when worn, the liner 1 can easily track the body of the wearer. Further, the moisture in the compression grooves 11 can escape through the liner 1 via the adhesive-free regions 1x to the outside. Therefore, a buildup of moisture between the wearer and the liner 1 is suppressed and dampness is suppressed.

Furthermore, in an embodiment according to the present invention, as explained with reference to FIG. 6, small sized SAP granules are arranged nearer to the skin-contact side region 6S, that is, the wearer. As a result, the moisture which moves through the compression grooves 11 is quickly removed by the small sized SAP granules and therefore dampness is reliably suppressed.

The liner 1 in some embodiments is, for example, packaged (e.g., for sale) in the form of an individual package. FIG. 8 shows one example of an individual package 1P of the liner 1. The liner 1 in this individual package 1P is, for example, folded along the areas around the above-mentioned defining lines DF and DR so that the skin contact side sheet 4 is positioned on the inside. One of the front region 6F and the rear region 6R is superposed on the other.

The individual package 1P is provided with a wrapping 12 covering substantially all of the outer surface of the liner 1 or the non-skin contact side sheet 5. The wrapping 12 is further provided with a moisture impermeable sheet 13 covering substantially all of the intermediate region 6I of the absorptive body 6. As a result, it is possible to maintain the hygroscopic performance of the hygroscopic material of the absorptive body 6, in particular, the hygroscopic material of the intermediate region 6I, until worn. Note that, one end of the wrapping 12 is fastened by a fastening piece 14 to another part of the wrapping 12 whereby the shape of the individual package 1P is maintained.

Here, the moisture impermeable sheet 13 is, for example, comprised of a sheet with a 24 hour moisture permeability of a value smaller than 100 g/m²·24 hrs in the case of conducting a moisture permeation test of JIS Z-0208 under a 40°C±0.5°C, 60%±2%RH environment.

In the moisture permeation test of JIS Z-208, pure water of 20 ml, measured by a micropipette, is poured into a reservoir (a cylindrical cup having an inner diameter of 60 mm). An opening of the reservoir is covered by a sample of a sheet to be tested, and is sealed securely with a sealing agent (a mixture of wax, etc., having a melting temperature of 60 to 70 °C and including 1.5 to 3 percent of oil). The reservoir is kept in a constant temperature and humidity device for 16 hours. Then, the weight (A) of the reservoir is measured. After that, the reservoir is again kept in the constant temperature and humidity device for 24 hours, and the weight (B) of the reservoir is again measured. The moisture permeability of the sample is calculated by dividing the cross-sectional area of the reservoir by the weight difference (A-B). The moisture permeability of the sheet is obtained by averaging the calculated moisture permeability of at least three samples.

This application claims the benefit of Japanese Application No. 2010-194203 the entire disclosure of which is incorporated by reference herein.

The present invention is also defined by way of the following non-limiting features Ea to Eg, E1 to E9 and U1 to U13, which are not specific to the detailed embodiments described above.

Ea a liner provided with adhesives applied to an outer surface of the non-skin contacting sheet and separated from each other in a width direction and extending in parallel in a longitudinal direction, the liner also being provided with compression grooves extending from said intermediate region up to said front region and rear region, said adhesives being positioned at the two sides around the front edges of said compression grooves in the width direction and extending over the front edges to the front in the longitudinal direction and being positioned at the two sides around the rear edges of said compression grooves in the width direction and extending over the rear edges to the rear in the longitudinal direction.

Eb a liner wherein said adhesives are provided so as not to be superposed around the front edges and around the rear edges of said compression grooves.

Ec a liner wherein said hygroscopic material is comprised of a high absorption polymer.

Ed a liner wherein the mass ratio of the high absorption polymer granules in said intermediate region which pass through a sieve of a mesh of 300 µm is higher than the mass ratio of the high absorption polymer granules in said front region which pass through a sieve of a mesh of 300 µm and higher than a mass ratio the high absorption polymer granules in said rear region which pass through a sieve of a mesh of 300 µm.

Ee a liner wherein the mass ratio of the high absorption polymer granules which pass through a sieve of a mesh of 300 µm in said absorptive body is at least 50 percent.

Ef a liner wherein the thickness of the absorptive body is divided into a skin contacting region and a non-skin contacting region and the mass ratio of the high absorption polymer granules which pass through a sieve of a mesh of 300 µm in said skin contacting region is higher than the mass ratio of the high absorption polymer granules which pass through a sieve of a mesh of 300 µm in said non-skin contacting region.

Eg a liner wherein said absorptive body includes an absorptive material in addition to said hygroscopic material, and a mass ratio of said hygroscopic material to said hygroscopic material and the absorptive material is 30 to 70 mass percent.

E1 An incontinence liner provided with:
a main body comprising a liquid permeable skin contacting sheet, a liquid impermeable and moisture permeable non-skin contacting sheet, and an absorptive body arranged between these skin contacting and non-skin contacting sheets, wherein said absorptive body includes a front region, a rear region, and an intermediate region between the front region and rear region; and
a pair of wings for fastening said liner to clothing, which wings being provided adjoining said intermediate region and being bent and superposed over said intermediate region when worn; wherein
said absorptive body includes a hygroscopic material; and
a basis weight of said hygroscopic material in said intermediate region is larger than a basis weight of the hygroscopic material at said front region and a basis weight of the hygroscopic material at said rear region.

E2 A liner as set forth in E1 wherein said liner is provided with adhesives applied to an outer surface of said non-skin contacting sheet and separated from each other in a width direction and extending in parallel in a longitudinal direction, and wherein said liner is provided with compression grooves extending from said intermediate region up to said front region and rear region, said adhesives being positioned at the two sides around the front edges of said compression grooves in the width direction and extending over the front edges to the front in the longitudinal direction and being positioned at the two sides around the rear edges of said compression grooves in the width direction and extending over the rear edges to the rear in the longitudinal direction.

E3 A liner as set forth in E1 or E2, wherein said adhesives are provided so as not to be superposed around the front edges and around the rear edges of said compression grooves.

E4 A liner as set forth in any one of E1 to E3, wherein said hygroscopic material is comprised of a high absorption polymer.

E5 A liner as set forth in E4, wherein the mass ratio of the high absorption polymer granules in said intermediate region which pass through a sieve of a mesh of 300 µm is higher than the mass ratio of the high absorption polymer granules in said front region which pass through a sieve of a mesh of 300 µm and higher than a mass ratio the high absorption polymer granules in said rear region which pass through a sieve of a mesh of 300 µm.

E6 A liner as set forth in E4 or E5, wherein the mass ratio of the high absorption polymer granules which pass through a sieve of a mesh of 300 µm in said absorptive body is at least 50 percent.

E7 A liner as set forth in any one of E4 to E6, wherein the thickness of said absorptive body is divided into a skin contacting region and a non-skin contacting region and the mass ratio of the high absorption polymer granules which pass through a sieve of a mesh of 300 µm in said skin contacting region is higher than the mass ratio of the high absorption polymer granules which pass through a sieve of a mesh of 300 µm in said non-skin contacting region.

E8 A liner as set forth in any one of E1 to E7, wherein said absorptive body includes an absorptive material in addition to said hygroscopic material, and a mass ratio of said hygroscopic material to said hygroscopic material and the absorptive material is 30 to 70 mass percent.

E9 An incontinence liner provided with:
a main body comprising a liquid permeable skin contacting sheet, a liquid impermeable and moisture permeable non-skin contacting sheet, and an absorptive body arranged between these skin contacting and non-skin contacting sheets, wherein said absorptive body includes a front region, a rear region, and an intermediate region between the front region and rear region; and
a pair of wings for fastening said liner to clothing, which wings being provided adjoining said intermediate region and being bent and superposed over said intermediate region when worn; wherein
said absorptive body includes a hygroscopic material; and
a basis weight of said hygroscopic material in said intermediate region is larger than a basis weight of the hygroscopic material at said front region and a basis weight of the hygroscopic material at said rear region;
said liner being provided with adhesives applied to an outer surface of said non-skin contacting sheet and separated from each other in a width direction and extending in parallel in a longitudinal direction, and with compression grooves extending from said intermediate region up to said front region and rear region,
said adhesives being positioned at the two sides around the front edges of said compression grooves in the width direction and extend over the front edges to the front in the longitudinal direction and are positioned at the two sides around the rear edges of said compression grooves in the width direction and extend over the rear edges to the rear in the longitudinal direction,
said adhesives being provided so as not to be superposed around the front edges and around the rear edges of said compression grooves,
said hygroscopic material being comprised of a high absorption polymer,
a mass ratio the high absorption polymer granules in said intermediate region which are able to pass through a sieve of a mesh of 300 µm being higher than a mass ratio of the high absorption polymer granules in said front region which are able to pass through a sieve of a mesh of 300 µm and a mass ratio of the high absorption polymer granules in said rear region which are able to pass through a sieve of a mesh of 300 µm,
the mass ratio of the high absorption polymer granules which are able to pass through a sieve of a mesh of 300 µm in said absorptive body being at least 50 percent,
said absorptive body including a skin contacting region and a non-skin contacting region wherein a mass ratio of the high absorption polymer granules which are able to pass through a sieve of a mesh of 300 µm is higher in said skin contacting region than in said non-skin contacting region, and
said absorptive body including an absorptive material in addition to said hygroscopic material, a mass ratio of said hygroscopic material to said hygroscopic material and the absorptive material being 30 to 70 mass percent.

U1 An incontinence liner, comprising:
a liquid permeable skin contact side sheet, a liquid impermeable and moisture permeable non-skin contact side sheet, and
an absorptive body arranged between these skin contact side sheet and non-skin contact side sheet, wherein
said absorptive body includes a front region, a rear region, and an intermediate region between these front region and rear region,
said liner is provided with a pair of wings for fastening said liner to clothing, said wings adjoining said intermediate region to be bent and superposed over said intermediate region when worn,
said absorptive body includes a hygroscopic material, and
a basis weight of said hygroscopic material in said intermediate region is larger than a basis weight of the hygroscopic material at said front region and a basis weight of the hygroscopic material at said rear region.

U2 A liner as set forth in U1, wherein said liner further comprises:
adhesives applied to an outer surface of said non-skin contact side sheet, separated from each other in a width direction, and extending in parallel in a longitudinal direction, and
compression grooves extending from said intermediate region up to said front region and rear region,
said adhesives being positioned at two sides around each of the front edges of each said compression groove in the width direction, and extending beyond the front edges toward the front in the longitudinal direction, and
said adhesives being positioned at two sides around each of the rear edges of each said compression groove in the width direction, and extending beyond the rear edges toward the rear in the longitudinal direction.

U3 A liner as set forth in U2, wherein said adhesives are provided without overlapping the front edges and the rear edges of said compression grooves.

U4 A liner as set forth in any one of U1 to U3, wherein said hygroscopic material is comprised of a high absorption polymer.

U5 A liner as set forth in U4, wherein
said high absorption polymer is in the form of high absorption polymer granules, and
a mass ratio of the part of the high absorption polymer granules passable through a sieve of a mesh of 300 µm in said intermediate region is higher than a mass ratio of the part of the high absorption polymer granules passable through a sieve of a mesh of 300 µm in said front region and a mass ratio of the part of the high absorption polymer granules passable through a sieve of a mesh of 300 µm in said rear region.

U6 A liner as set forth in U4 or U5, wherein the mass ratio of the part of the high absorption polymer granules passable through a sieve of a mesh of 300 µm in said absorptive body is at least 50 percent.

U7 A liner as set forth in any one of U4 to U6, wherein
said absorptive body includes a skin contact side region and a non-skin contact side region, and
a mass ratio of the part of the high absorption polymer granules passable through a sieve of a mesh of 300 µm in said skin contact side region is higher than a mass ratio of the part of the high absorption polymer granules passable through a sieve of a mesh of 300 µm in said non-skin contact side region.

U8 A liner as set forth in any one of U1 to U7, wherein said absorptive body includes an absorptive material in addition to said hygroscopic material, and a mass ratio of (i) said hygroscopic material to (ii) said hygroscopic material and the absorptive material is 30 to 70 mass percent.

U9 An incontinence liner, comprising:
a liquid permeable skin contact side sheet,
a liquid impermeable and moisture permeable non-skin contact side sheet, and
an absorptive body arranged between these skin contact side sheet and non-skin contact side sheet,
wherein
said absorptive body includes a front region, a rear region, and an intermediate region between these front region and rear region,
said liner is provided with a pair of wings for fastening said liner to clothing, said wings adjoining said intermediate region to sandwich the clothing and to be superposed over said intermediate region when worn,
said absorptive body includes a hygroscopic material,
a basis weight of said hygroscopic material in said intermediate region is larger than a basis weight of the hygroscopic material at said front region and a basis weight of the hygroscopic material at said rear region,
said liner further comprises:
adhesives applied to an outer surface of said non-skin contact side sheet, separated from each other in a width direction, and extending in parallel in a longitudinal direction, and
compression grooves extending from said intermediate region up to said front region and rear region,
said adhesives are positioned at two sides around each of the front edges of each said compression groove in the width direction, and extend beyond the front edges toward the front in the longitudinal direction,
said adhesives are positioned at two sides around each of the rear edges of each said compression groove in the width direction, and extend beyond the rear edges toward the rear in the longitudinal direction,
said adhesives are provided without overlapping the front edges and the rear edges of said compression grooves,
said hygroscopic material is comprised of high absorption polymer granules,
a mass ratio of the part of the high absorption polymer granules passable through a sieve of a mesh of 300 µm in said intermediate region is higher than a mass ratio of the part of the high absorption polymer granules passable through a sieve of a mesh of 300 µm in said front region and a mass ratio of the part of the high absorption polymer granules passable through a sieve of a mesh of 300 µm in said rear region,
the mass ratio of the part of the high absorption polymer granules passable through a sieve of a mesh of 300 µm in said absorptive body is at least 50 percent,
said absorptive body includes a skin contact side region and a non-skin contact side region,
a mass ratio of the part of the high absorption polymer granules passable through a sieve of a mesh of 300 µm in said skin contact side region is higher than a mass ratio of the part of the high absorption polymer granules passable through a sieve of a mesh of 300 µm in said non-skin contact side region, and
said absorptive body includes an absorptive material in addition to said hygroscopic material, and a mass ratio of (i) said hygroscopic material to (ii) said hygroscopic material and the absorptive material is 30 to 70 mass percent.

U10 An individual incontinence liner package, comprising:
an incontinence liner as set forth in U1, and
a moisture impermeable sheet wrapping around the incontinence liner for maintaining a hygroscopic performance of the hygroscopic material of the absorptive body of the liner until the liner is worn.

U11 An individual incontinence liner package as set forth in U10, wherein
the packaged liner is folded along front and rear dividing lines between the intermediate region and the front region and between the intermediate region and the rear region, respectively.

U12 An individual incontinence liner package as set forth in U11, wherein
the skin contact side sheet is positioned on the inside in the folded liner.

U13 An individual incontinence liner package as set forth in U11, further comprising:
a fastening piece fastening one end of the moisture impermeable sheet to another part of the moisture impermeable sheet whereby the liner is maintained in the folded state.

### Reference Signs List

- 1: incontinence liner
- 2: main body
- 3: wings
- 4: skin contact side sheet
- 5: non-skin contact side sheet
- 6: absorptive body
- 9, 10: adhesive
- 11: compression grooves
- 12: wrapping
- 13: moisture impermeable sheet

## Claims

1. An incontinence liner (1) provided with a liquid permeable skin contact side sheet (4), a liquid impermeable and moisture permeable non-skin contact side sheet (5), and an absorptive body (6) arranged between these skin contact side sheet (4) and non-skin contact side sheet (5), in which liner,
said absorptive body (6) includes a front region (6F), a rear region (6R), and an intermediate region (6I) between these front region (6F) and rear region (6R),
said liner (1) is provided with a pair of wings (3) for fastening said liner to clothing, which wings (3) being provided adjoining said intermediate region (6I) and being bent and superposed over said intermediate region (6I) when worn,
said absorptive body (6) includes a hygroscopic material, and
a basis weight of said hygroscopic material in said intermediate region (6I) is larger than a basis weight of the hygroscopic material at said front region and a basis weight of the hygroscopic material at said rear region (6R),
wherein said liner (1) is provided with adhesives (9) applied to an outer surface of said non-skin contact side sheet (5) and extending separated from each other in a width direction, and extending in parallel in a longitudinal direction and compression grooves (11) extending from said intermediate region (6I) up to said front region (6F) and rear region (6R), said adhesives (9) being positioned at the two sides around the front edges (11F) of said compression grooves (11) in the width direction and extending beyond the front edges (11F) toward the front in the longitudinal direction and being positioned at the two sides around the rear edges (11R) of said compression grooves (11) in the width direction and extending over the rear edges (11R) to the rear in the longitudinal direction.

2. A liner as set forth in claim 1, wherein said adhesives (9) are provided so as not to be superposed around the front edges (11F) and around the rear edges (11R) of said compression grooves (11).

3. A liner as set forth in claim 1 or 2, wherein said hygroscopic material is comprised of a high absorption polymer.

4. A liner as set forth in claim 3, wherein the mass ratio of the part of the high absorption polymer granules passable through a sieve of a mesh of 300 µm in said intermediate region is higher than a mass ratio of the part of the high absorption polymer granules passable through a sieve of a mesh of 300 µm in said front region and a mass ratio of the part of the high absorption polymer granules passable through a sieve of a mesh of 300 µm in said rear region.

5. A liner as set forth in claim3 or 4, wherein the mass ratio of the part of the high absorption polymer granules passable through a sieve of a mesh of 300 µm in said absorptive body is at least 50 percent.

6. A liner as set forth in any one of claims 3 to 5, wherein said absorptive body (6) includes a skin contact side region (6S) and a non-skin contact side region (6NS) and a mass ratio of the part of the high absorption polymer granules passable through a sieve of a mesh of 300 µm in said skin contact side region is higher than a mass ratio of the part of the high absorption polymer granules passable through a sieve of a mesh of 300 µm in said non-skin contact side region.

7. A liner as set forth in any one of claims 1 to 6, wherein said absorptive body (6) includes an absorptive material in addition to said hygroscopic material, and a mass ratio of said hygroscopic material to said hygroscopic material and the absorptive material is 30 to 70 mass percent.

8. A liner as set forth in claim 1, wherein the pair of wings for fastening said liner to clothing sandwich clothing when worn,
said adhesives (9) are provided so as not to be superposed around the front edges (11F) and around the rear edges (11R) of said compression grooves (11),
said hygroscopic material is comprised of a high absorption polymer granules,
a mass ratio of the part of the high absorption polymer granules passable through a sieve of a mesh of 300 µm in said intermediate region (6I) is higher than a mass ratio of the part of the high absorption polymer granules passable through a sieve of a mesh of 300 µm in said front region (6F) and a mass ratio of the part of the high absorption polymer granules passable through a sieve of a mesh of 300 µm in said rear region (6R),
the mass ratio of the part of the high absorption polymer granules passable through a sieve of a mesh of 300 µm in said absorptive body (6) is at least 50 percent,
said absorptive body (6) includes a skin contact side region (6S) and a non-skin contact side region (6NS) and a mass ratio of the part of the high absorption polymer granules passable through a sieve of a mesh of 300 µm in said skin contact side region is higher than a mass ratio of the part of the high absorption polymer granules passable through a sieve of a mesh of 300 µm in said non-skin contact side region, and
said absorptive body (6) includes an absorptive material in addition to said hygroscopic material, and a mass ratio of said hygroscopic material to said hygroscopic material and the absorptive material is 30 to 70 mass percent.

## Patentansprüche

1. Inkontinenzeinlage (1), die mit Folgendem bereitgestellt ist: einer flüssigkeitsdurchlässigen Lage auf der Hautkontaktseite (4), einer flüssigkeitsundurchlässigen und feuchtigkeitsdurchlässigen Lage auf der Nicht-Hautkontaktseite (5) und einem absorbierenden Körper (6), der zwischen dieser Lage auf der Hautkontaktseite (4) und Lage auf der Nicht-Hautkontaktseite (5) angeordnet ist, in welcher Einlage
der absorbierende Körper (6) eine vordere Region (6F), eine hintere Region (6R) und eine dazwischenliegende Region (61) zwischen dieser vorderen Region (6F) und hinteren Region (6R) einschließt,
die Einlage (1) mit einem Paar Flügel (3) zur Befestigung der Einlage an Kleidung bereitgestellt ist, welche Flügel (3) angrenzend an die dazwischenliegende Region (61) und gebogen und überlagert über die dazwischenliegende Region (61) beim Tragen bereitgestellt sind,
der absorbierende Körper (6) ein hygroskopisches Material einschließt und ein Basisgewicht des hygroskopischen Materials in der dazwischenliegenden Region (61) größer ist als ein Basisgewicht des hygroskopischen Materials an der vorderen Region und ein Basisgewicht des hygroskopischen Materials an der hinteren Region (6R),
wobei die Einlage (1) mit Folgendem bereitgestellt ist: Haftmitteln (9), die auf eine äußere Oberfläche der Lage auf der Nicht-Hautkontaktseite (5) aufgetragen sind und sich getrennt voneinander in einer Breitenrichtung erstrecken und sich parallel in einer Längsrichtung erstrecken, und Kompressionsrillen (11), die sich von der dazwischenliegenden Region (61) bis zu der vorderen Region (6F) und hinteren Region (6R) erstrecken, wobei die Haftmittel (9) an den zwei Seiten um die vorderen Ränder (11F) der Kompressionsrillen (11) in der Breitenrichtung positioniert sind und sich über die vorderen Ränder (11 F) zur Vorderseite in der Längsrichtung hinaus erstrecken und an den zwei Seiten um die hinteren Ränder (11R) der Kompressionsrillen (11) in der Breitenrichtung positioniert sind und sich über die hinteren Ränder (11R) zur Hinterseite in der Längsrichtung erstrecken.

2. Einlage wie in Anspruch 1 dargelegt, wobei die Haftmittel (9) derart bereitgestellt sind, dass sie nicht um die vorderen Ränder (11F) und um die hinteren Ränder (11R) der Kompressionsrillen (11) überlagert sind.

3. Einlage wie in Anspruch 1 oder 2 dargelegt, wobei das hygroskopische Material aus einem hochabsorbierenden Polymer besteht.

4. Einlage wie in Anspruch 3 dargelegt, wobei der Massenanteil des Teils des hochabsorbierenden Polymergranulats, das durch ein Sieb einer Maschenweite von 300 µm passierbar ist, in der dazwischenliegenden Region höher ist als ein Massenanteil des Teils des hochabsorbierenden Polymergranulats, das durch ein Sieb einer Maschenweite von 300 µm passierbar ist, in der vorderen Region und ein Massenanteil des Teils des hochabsorbierenden Polymergranulats, das durch ein Sieb einer Maschenweite von 300 µm passierbar ist, in der hinteren Region.

5. Einlage wie in Anspruch 3 oder 4 dargelegt, wobei der Massenanteil des Teils des hochabsorbierenden Polymergranulats, das durch ein Sieb einer Maschenweite von 300 µm passierbar ist, in dem absorbierenden Körper mindestens 50 Prozent beträgt.

6. Einlage wie in einem der Ansprüche 3 bis 5 dargelegt, wobei der absorbierende Körper (6) eine Region auf der Hautkontaktseite (6S) und eine Region auf der Nicht-Hautkontaktseite (6NS) einschließt und ein Massenanteil des Teils des hochabsorbierenden Polymergranulats, das durch ein Sieb einer Maschenweite von 300 µm passierbar ist, in der Region auf der Hautkontaktseite höher ist als ein Massenanteil des Teils des hochabsorbierenden Polymergranulats, das durch ein Sieb einer Maschenweite von 300 µm passierbar ist, in der Region auf der Nicht-Hautkontaktseite.

7. Einlage wie in einem der Ansprüche 1 bis 6 dargelegt, wobei der absorbierende Körper (6) ein absorbierendes Material zusätzlich zu dem hygroskopischen Material einschließt und ein Masseverhältnis des hygroskopischen Materials zu dem hygroskopischen Material und dem absorbierenden Material 30 bis 70 Masseprozent beträgt.

8. Einlage wie in Anspruch 1 dargelegt, wobei das Paar Flügel zur Befestigung der Einlage an Kleidung beim Tragen Kleidung dazwischenklemmt,
die Haftmittel (9) derart bereitgestellt sind, dass sie nicht um die vorderen Ränder (11F) und um die hinteren Ränder (11R) der Kompressionsrillen (11) überlagert sind,
das hygroskopische Material aus einem hochabsorbierenden Polymergranulat besteht,
ein Massenanteil des Teils des hochabsorbierenden Polymergranulats, das durch ein Sieb einer Maschenweite von 300 µm passierbar ist, in der dazwischenliegenden Region (61) höher ist als ein Massenanteil des Teils des hochabsorbierenden Polymergranulats, das durch ein Sieb einer Maschenweite von 300 µm passierbar ist, in der vorderen Region (6F) und ein Massenanteil des Teils des hochabsorbierenden Polymergranulats, das durch ein Sieb einer Maschenweite von 300 µm passierbar ist, in der hinteren Region (6R),
der Massenanteil des Teils des hochabsorbierenden Polymergranulats, das durch ein Sieb einer Maschenweite von 300 µm passierbar ist, in dem absorbierenden Körper (6) mindestens 50 Prozent beträgt,
der absorbierende Körper (6) eine Region auf der Hautkontaktseite (6S) und eine Region auf der Nicht-Hautkontaktseite (6NS) einschließt und ein Massenanteil des Teils des hochabsorbierenden Polymergranulats, das durch ein Sieb einer Maschenweite von 300 µm passierbar ist, in der Region auf der Hautkontaktseite höher ist als ein Massenanteil des Teils des hochabsorbierenden Polymergranulats, das durch ein Sieb einer Maschenweite von 300 µm passierbar ist, in der Region auf der Nicht-Hautkontaktseite, und
der absorbierende Körper (6) ein absorbierendes Material zusätzlich zu dem hygroskopischen Material einschließt und ein Masseverhältnis des hygroskopischen Materials zu dem hygroskopischen Material und dem absorbierenden Material 30 bis 70 Masseprozent beträgt.

## Revendications

1. Garniture de protection contre l'incontinence (1) comportant une feuille côté en contact avec la peau (4) qui est perméable aux liquides, une feuille côté non en contact avec la peau (5) qui est imperméable aux liquides et perméable à l'humidité, et un corps absorbant (6) agencé entre ces feuille côté en contact avec la peau (4) et feuille côté non en contact avec la peau (5), garniture dans laquelle,
ledit corps absorbant (6) comprend une région avant (6F), une région arrière (6R), et une région intermédiaire (6I) entre ces région avant (6F) et région arrière (6R),
ladite garniture (1) comporte une paire d'ailettes (3) servant à attacher ladite garniture au vêtement, ailettes (3) qui sont mises en oeuvre de manière contiguë par rapport à ladite région intermédiaire (6I) et qui sont pliées et superposées sur ladite région intermédiaire (61) quand la garniture est portée,
ledit corps absorbant (6) comprend un matériau hygroscopique, et
une masse surfacique dudit matériau hygroscopique dans ladite région intermédiaire (6I) est supérieure par rapport à une masse surfacique du matériau hygroscopique au niveau de ladite région avant et à une masse surfacique du matériau hygroscopique au niveau de ladite région arrière (6R),
dans laquelle ladite garniture (1) comporte des éléments adhésifs (9) appliqués sur une surface extérieure de ladite feuille côté non en contact avec la peau (5) et s'étendant de manière séparée les uns par rapport aux autres dans une direction allant dans le sens de la largeur, et s'étendant de manière parallèle dans une direction allant dans le sens longitudinal et des rainures de compression (11) s'étendant depuis ladite région intermédiaire (6I) jusqu'à ladite région avant (6F) et ladite région arrière (6R), lesdits éléments adhésifs (9) étant positionnés au niveau des deux côtés autour des bords avant (11F) desdites rainures de compression (11) dans la direction allant dans le sens de la largeur et s'étendant au-delà des bords avant (11F) vers la partie avant dans la direction allant dans le sens longitudinal et étant positionnés au niveau des deux côtés autour des bords arrière (11R) desdites rainures de compression (11) dans la direction allant dans le sens de la largeur et s'étendant par-dessus les bords arrière (11R) vers l'arrière dans la direction allant dans le sens longitudinal.

2. Garniture selon la revendication 1, dans laquelle lesdits éléments adhésifs (9) sont mis en oeuvre de manière à ne pas être superposés autour des bords avant (11F) et autour des bords arrière (11R) desdites rainures de compression (11).

3. Garniture selon la revendication 1 ou la revendication 2, dans laquelle ledit matériau hygroscopique est constitué d'un polymère à haute absorption.

4. Garniture selon la revendication 3, dans laquelle le rapport de masse de la partie des granules de polymère à haute absorption en mesure de passer au travers d'un crible d'une maille de 300 µm dans ladite région intermédiaire est supérieur par rapport à un rapport de masse de la partie des granules de polymère à haute absorption en mesure de passer au travers d'un crible d'une maille de 300 µm dans ladite région avant et à un rapport de masse de la partie des granules de polymère à haute absorption en mesure de passer au travers d'un crible d'une maille de 300 µm dans ladite région arrière.

5. Garniture selon la revendication 3 ou la revendication 4, dans laquelle le rapport de masse de ladite partie des granules de polymère à haute absorption en mesure de passer au travers d'un crible d'une maille de 300 µm dans ledit corps absorbant est au moins de 50 pour cent.

6. Garniture selon l'une quelconque des revendications 3 à 5, dans laquelle ledit corps absorbant (6) comprend une région côté en contact avec la peau (6S) et une région côté non en contact avec la peau (6NS) et un rapport de masse de la partie des granules de polymère à haute absorption en mesure de passer au travers d'un crible d'une maille de 300 µm dans ladite région côté en contact avec la peau est supérieur par rapport à un rapport de masse de la partie des granules de polymère à haute absorption en mesure de passer au travers d'un crible d'une maille de 300 µm dans ladite région côté non en contact avec la peau.

7. Garniture selon l'une quelconque des revendications 1 à 6, dans laquelle ledit corps absorbant (6) comprend un matériau absorbant en plus dudit matériau hygroscopique, et un rapport de masse dudit matériau hygroscopique par rapport audit matériau hygroscopique et audit matériau absorbant est compris entre 30 et 70 pour cent en masse.

8. Garniture selon la revendication 1, dans laquelle la paire d'ailettes servant à attacher ladite garniture au vêtement prennent le vêtement en sandwich quand la garniture est portée,
lesdits éléments adhésifs (9) sont mis en oeuvre de manière à ne pas être superposés autour des bords avant (11F) et autour des bords arrière (11R) desdites rainures de compression (11),
ledit matériau hygroscopique est constitué de granules de polymère à haute absorption,
un rapport de masse de la partie des granules de polymère à haute absorption en mesure de passer au travers d'un crible d'une maille de 300 µm dans ladite région intermédiaire (6I) est supérieur par rapport à un rapport de masse de la partie des granules de polymère à haute absorption en mesure de passer au travers d'un crible d'une maille de 300 µm dans ladite région avant (6F) et à un rapport de masse de la partie des granules de polymère à haute absorption en mesure de passer au travers d'un crible d'une maille de 300 µm dans ladite région arrière (6R),
le rapport de masse de ladite partie des granules de polymère à haute absorption en mesure de passer au travers d'un crible d'une maille de 300 µm dans ledit corps absorbant (6) est au moins de 50 pour cent,
ledit corps absorbant (6) comprend une région côté en contact avec la peau (6S) et une région côté non en contact avec la peau (6NS) et un rapport de masse de la partie des granules de polymère à haute absorption en mesure de passer au travers d'un crible d'une maille de 300 µm dans ladite région côté en contact avec la peau est supérieur par rapport à un rapport de masse de la partie des granules de polymère à haute absorption en mesure de passer au travers d'un crible d'une maille de 300 µm dans ladite région côté non en contact avec la peau, et
ledit corps absorbant (6) comprend un matériau absorbant en plus dudit matériau hygroscopique, et un rapport de masse dudit matériau hygroscopique par rapport audit matériau hygroscopique et audit matériau absorbant est compris entre 30 et 70 pour cent en masse.
